# EUROPEAN PATENT APPLICATION

(11) **EP 4 057 001 A1**
(43) Date of publication of application: **14.09.2022**
(21) Application number: 20896682.0
(22) Date of filing: 04.12.2020
(51) Int. Cl.: G01N 33/50, C12M 3/06, C12M 1/00, B01L 3/00, C12N 5/0793, C12N 5/079

(54) **BIOMIMETIC NERVE CHIP FOR EVALUATING EFFICACY AND TOXICITY ON NERVE, AND USE THEREOF**

(30) Priority: 05.12.2019 KR 20190160376
(71) Applicant: Cha University Industry-Academic Cooperation Foundation, Pocheon-si, Gyeonggi-do 11160 (KR)
(72) Inventor: KO, Sungho, Suwon-si Gyeonggi-do 16514 (KR); CHOI, Geonho, Seongnam-si Gyeonggi-do 13420 (KR)
(74) Representative: Gassner, Birgitta
(86) International application number: PCT/KR2020/017696
(87) International publication number: WO 2021/112638

(57) **Abstract**

The present invention relates to a biomimetic nerve chip for evaluating the efficacy and toxicity of a drug, a method for evaluating the efficacy of a drug on nerve cells through astrocytes by using the biomimetic nerve chip, and a method for evaluating the toxicity of a drug on nerve cells through astrocytes by using the biomimetic nerve chip, the biomimetic nerve chip comprising: an astrocyte supply unit and a nerve cell supply unit for simulating nerve tissue; and a culture solution supply unit for supplying a culture solution to the astrocyte supply unit and the nerve cell supply unit. By using the biomimetic nerve chip for evaluating the efficacy and toxicity of a drug provided in the present invention, it is possible to overcome inaccuracies due to differences between the different species in animal experiments in the study of nerve tissues, and using a combination of astrocytes and nerve cells enables use of the nerve chip as a platform to more accurately evaluate the efficacy and toxicity of a drug under conditions similar to *in vivo* conditions, and the nerve chip can be applied to studies of microenvironments in nerve tissues and other organ-on-a-chip studies. Therefore, the present invention may be utilized in the development of a human-on-a-chip that can effectively analyze the efficacy and toxicity of a drug.

## Description

### Technical Field

The present invention relates to a biomimetic nerve chip for evaluating efficacy and toxicity on the nerve and use thereof, and more specifically to a biomimetic nerve chip for evaluating efficacy and toxicity of a drug on neurons, the biomimetic nerve chip including: an astrocyte supply part and a neuron supply part for simulating the nervous tissue; and a culture medium supply part for supplying a culture medium to the astrocyte supply part and the neuron supply part, to a method for evaluating efficacy of a drug on neurons through astrocytes by using the biomimetic nerve chip, and to a method for evaluating toxicity of a drug on neurons through astrocytes by using the biomimetic nerve chip.

### Background Art

Despite increasing social, clinical, and economic demands for therapies targeting neurological diseases, development of related drugs has been slow since 1990, and clinical trials therefor are known to be more likely to fail than those for other types of drugs. Although toxicity evaluation through epidemiological studies among toxicity studies is known to be most reliable, these methods require much money and time. Therefore, drug evaluation using various species of animals, such as rats, mice, guinea pigs, and rabbits, was conducted to evaluate toxicity within a relatively short time, but this was also not free from ethical issues, and in many cases, animal testing results cannot be applied in the same manner to humans due to the limitations of inter-species differences, such as the existence of different ion channels from humans and pharmacokinetic differences, and even drugs that have passed clinical trials through animal testing are often withdrawn due to side effects thereof. The development of new drugs through animal testing requires a long period of 10-15 years and a high cost of about 5 trillion Korean won, and despite such inefficiency, animal testing is still being employed since there is currently no way to replace animal testing. Hence, there is an urgent need for development of more efficient toxicity evaluation systems capable of replacing animal testing.

Organ-on-a-chip (OOC) has recently attracted attention as technology that can more accurately evaluate toxicity *in vitro* in place of animal testing. This can mimic an internal microenvironment structure of a specific organ to culture cells constituting the corresponding organ, thereby implementing functions and characteristics of the organ. This is expected as a platform that can replace existing drug development and toxicity evaluation methods, wherein instead of simply culturing cells on a chip, on the basis of the fact that the movement and absorption of oxygen and nutrients, the movement of signal transmitters, and the like occur on the scale of micrometers to millimeters, a micrometer-level microenvironment in the human body tissue is implemented by soft-lithography developed from microfluidic technology and semiconductor micro-processing, and living cells are cultured in micro-chambers connected by a continuous perfusion system, and these chambers are then arranged to implement tissue- or organ-level physiological activity.

However, organ biomimetic chips are in the initial research stage, and thus have a number of limitations to be overcome, such as a failure to accurately reflect an actual *in vivo* mechanism in previous studies. Previous studies by Professor Donna J. Webb's research team published in 2011 and 2013, which are similar to the present invention and referenced for the development of biomimetic nerve chips, are directed to microfluidic platforms in which animal-derived neurons and astrocytes are selectively injected into a neuron chamber and an astrocyte chamber divided by an open/close-type micro-valve using PDMS, followed by co-culture. Related studies were significant as being conducted in the early stage, but left much to be desired, such as animal-derived cells being used, selective injection being performed using a relatively complex actuated micro-valve, cells in chambers being exposed to strong shear stress, and channel design being unsatisfactory.

Additionally, neuro-biomimetic chips, which have been previously studied for drug evaluation targeting a nervous system, are studied mainly focusing on mimicking methods of the blood-brain barrier (BBB). For example, Korean Patent Publication No. 10-2019-0044630 discloses an *in vitro* blood-brain barrier model including an endothelial cell layer and a brain tissue layer composed of neurons and optionally one or more astrocytes, pericytes, oligodendrocytes, and microglia. However, such a model has technical limitations in mimicking up to the *in vivo* BBB function level, and even when the brain barrier system is not damaged, there is still the possibility that toxic substances will pass through cell membranes with non-specific permeability and through claudin-2, which is present at the tight junction of BCSFB and OBB, except for BBB, among the types of the brain barrier. Therefore, despite the importance of BBB research, it is considered to be a more realistic research direction to precede studies about an accurate situation after the penetration of toxic substances into the brain tissue rather than to mimic an unstable brain barrier system.

There have been several other attempts to bio-mimic the nervous system; and for example, U.S. Patent No. 7403883 discloses a three-dimensional *in vitro* spine model mimicking a human spine, the model including: a model body defining a curved passageway in the shape of a human spinal canal; and an artificial cord structure located and fixed within the passageway to simulate a human spinal cord structure, and Korean Patent No. 10-0994451 discloses a biomimetic neuron circuit composed of variable negative resistors and having arithmetic functions, wherein biomimetic neurons and synapses are implemented by variable negative resistors and trans-conductors to have pulse signal generation and signal transmission characteristics suitable for nerve chip design.

Since physiological phenomena in the human body, especially physiological phenomena occurring in the organ level, are composed of very complex and systematic systems, there are currently technical limitations in mimicking all physiological phenomena. Therefore, identifying a systemic mechanism by selecting some essential physiological characteristics and observing the characteristics in various aspects to ultimately implement a comprehensive platform is a good method for understanding a mechanism for a specific physiological phenomenon *in vivo.*

### Disclosure

### Technical Problem

The present inventors have made intensive research efforts to develop a method for implementing neurons at a similar level to the nervous system *in vitro,* and as a result, the present inventors confirmed that the use of a biomimetic nerve chip, which includes an astrocyte supply part and a neuron supply part mimicking the nervous tissue and a culture medium supply part for supplying a culture medium to the astrocyte supply part and the neuron supply part, enables the observation of cell response changes of neurons and astrocytes, thereby allowing easy evaluation of efficacy and toxicity of a drug on each type of cells.

### Technical Solution

An aspect of the present invention is to provide a biomimetic nerve chip for evaluating efficacy and toxicity of a drug, the biomimetic nerve chip including an astrocyte supply part and a neuron supply part for mimicking the nervous tissue and a culture medium supply part for supplying a culture medium to the astrocyte supply part and the neuron supply part.

Another aspect of the present invention is to provide a method for evaluating efficacy of a drug on neurons through astrocytes by using the biomimetic nerve chip.

Still another aspect of the present invention is to provide a method for evaluating toxicity of a drug on neurons through astrocytes by using the biomimetic nerve chip.

Still another aspect of the present invention is to provide use of a biomimetic nerve chip for evaluating toxicity of a drug on neurons.

### Advantageous Effects

The use of the biomimetic nerve chip for evaluating efficacy and toxicity of a drug provided in the present invention can overcome inaccuracies due to inter-species differences in the animal testing with respect to studies of nervous tissues, can be used for a platform that evaluate more accurately the efficacy and toxicity of a drug under conditions similar to *in vivo* conditions by using a combination of astrocytes and neurons, and can be applied to studies of micro-environments in the nervous tissues and other organ-on-a-chip. Therefore, the present invention may be utilized in the development of a human-on-a-chip that can effectively analyze the efficacy and toxicity of a drug.

### Brief Description of Drawings

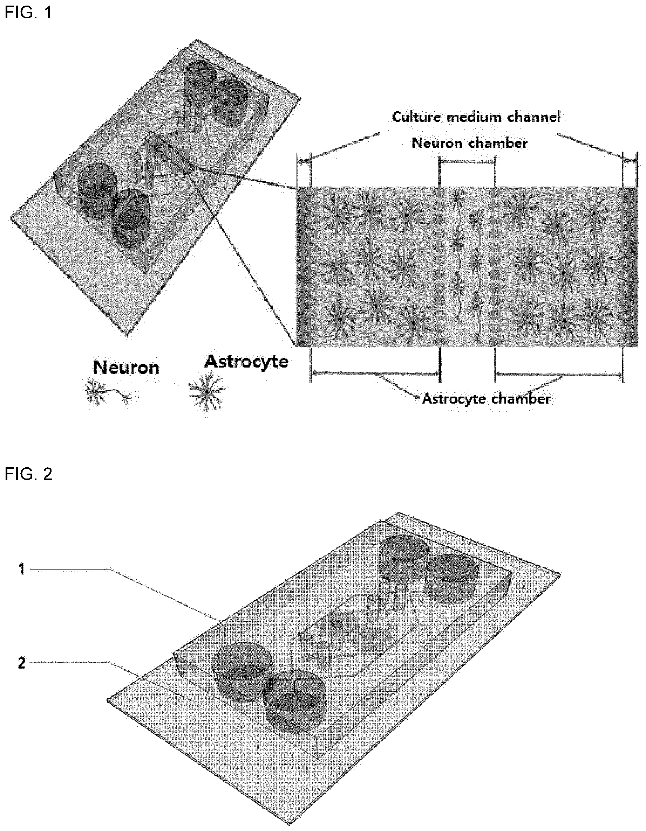

FIG. 1 illustrates a three-dimensional view of a biomimetic nerve chip of the present invention and an overall schematic view including elements in the chip.
FIG. 2 illustrates a three-dimensional view of the biomimetic nerve chip of the present invention.

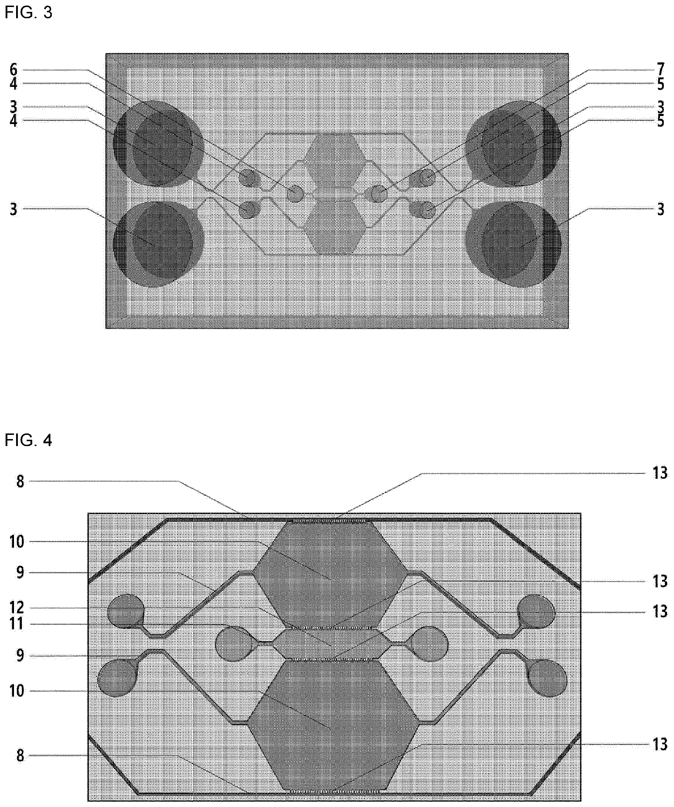

FIG. 3 illustrates a plane view of a microfluidic device of the biomimetic nerve chip of the present invention.
FIG. 4 illustrates an enlarged view of neuron chambers and channels, astrocyte chambers and channels, and culture medium channels of the microfluidic device in the biomimetic nerve chip of the present invention.

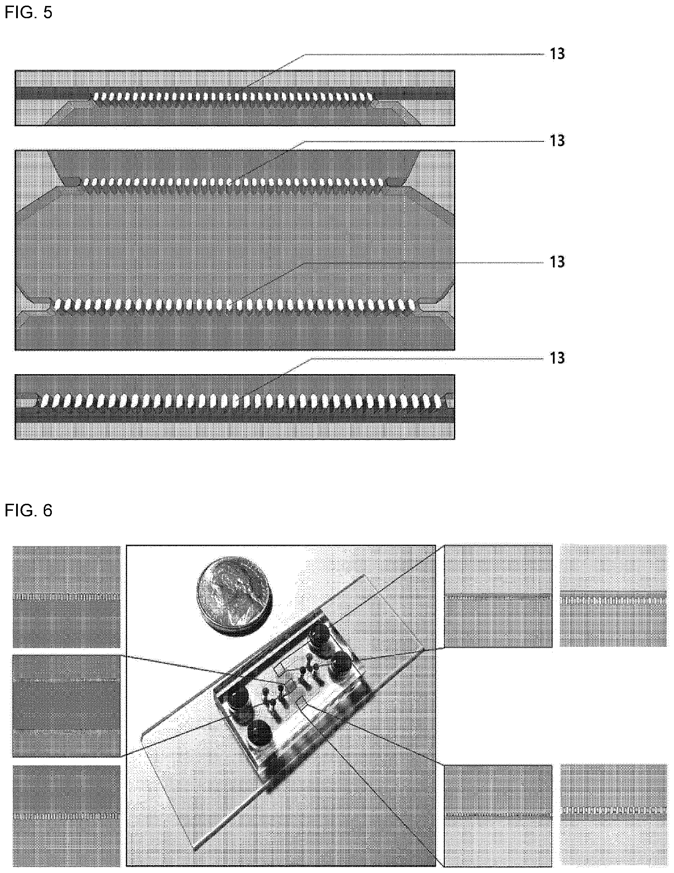

FIG. 5 illustrates an enlarged view of micro-post structures of the microfluidic device in the biomimetic nerve chip of the present invention.
FIG. 6 illustrates an actual image of the biomimetic nerve chip of the present invention and microscopic images confirming the possibility of selective separation injection by injecting food colorings into each chamber and channel in the chip.

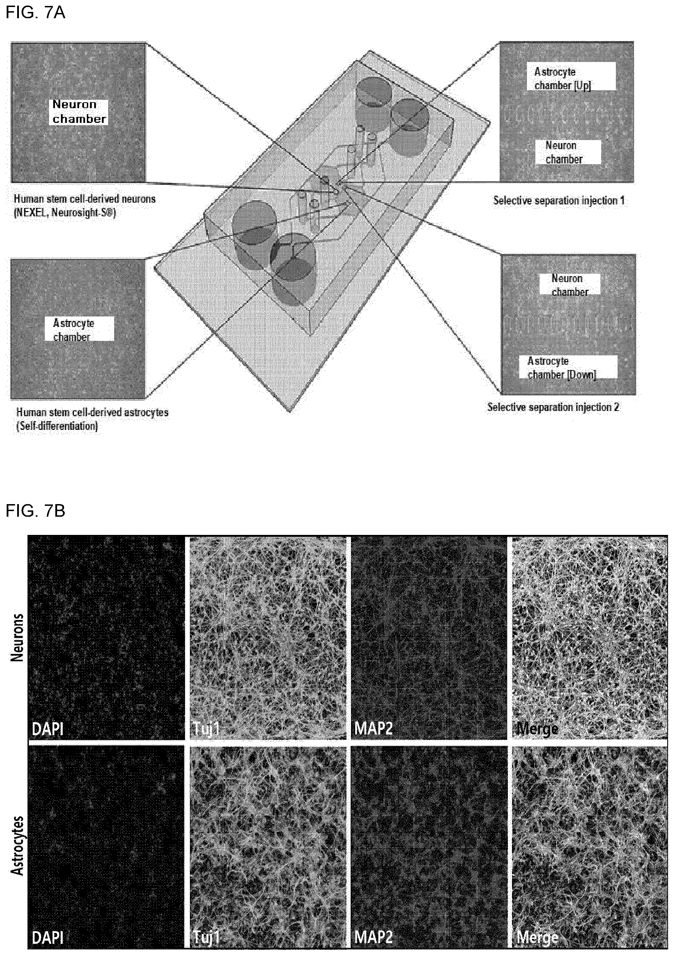

FIG. 7A illustrates microscopic images confirming the possibility of co-culture of neurons and astrocytes and the possibility of selective separation injection of corresponding cells.
FIG. 7B illustrates confocal microscopic images confirming whether neurons and astrocytes were co-cultured in a healthy state in the biomimetic nerve chip of the present invention, through immunostaining using antibodies used as specific markers for different types of cells.

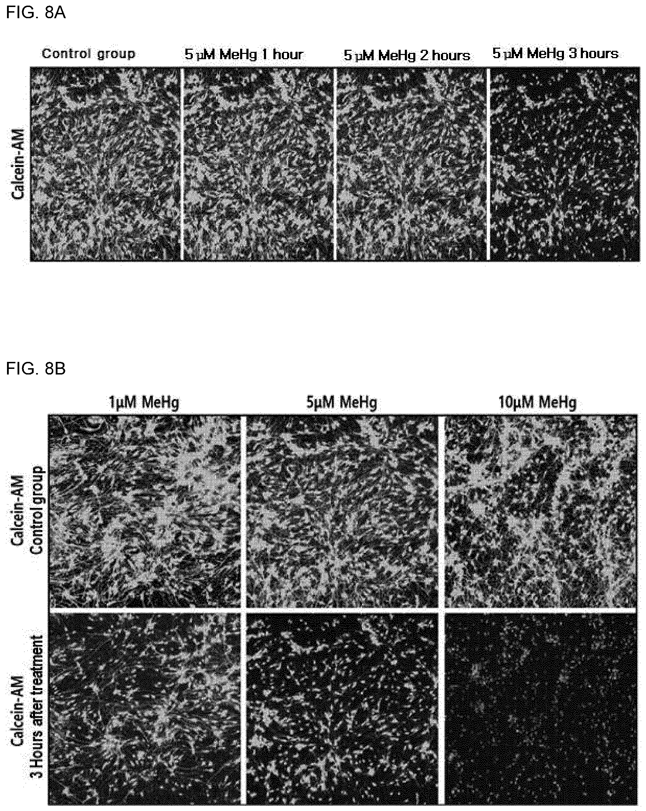

FIG. 8A illustrates confocal microscopic images obtained when only neurons were cultured alone for 3 days in the biomimetic nerve chip of the present invention, treated with MeHg, a substance inducing neurotoxicity, at a concentration of 5 µM, and then stained with Calcein-AM to observe viability and morphological changes at 1-hour intervals for 3 hours.
FIG. 8B illustrates confocal microscopic images obtained when only neurons were cultured alone for 3 days in the biomimetic nerve chip of the present invention, treated with MeHg, a substance inducing neurotoxicity, at concentrations of 1 µM, 5 µM, and 10 µM, and then stained with Calcein-AM to observe viability and morphological changes thereof according to the concentration after 3 hours.

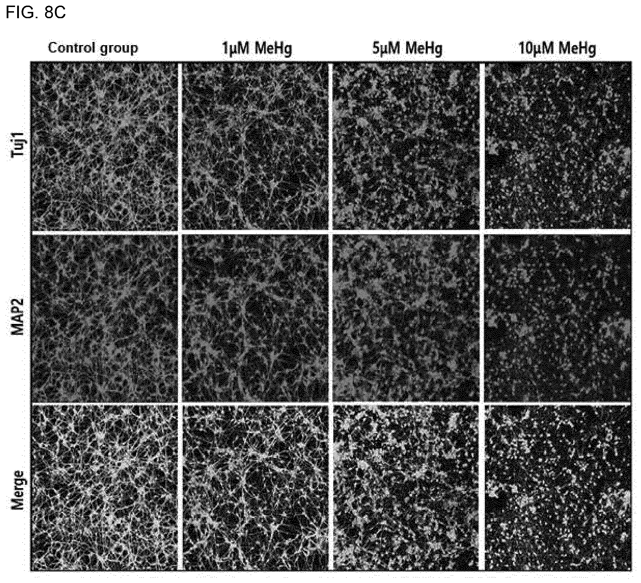

FIG. 8C illustrates confocal microscopic images obtained when only neurons were cultured alone for 3 days in the biomimetic nerve chip of the present invention, treated with MeHg, a substance inducing neurotoxicity, at concentrations of 1 µM, 5 µM, and 10 µM, and then stained with the neuron-specific markers Tuj1 and MAP2 to confirm the identification thereof and observe morphological and internal protein expression changes thereof according to the concentration after 3 hours.

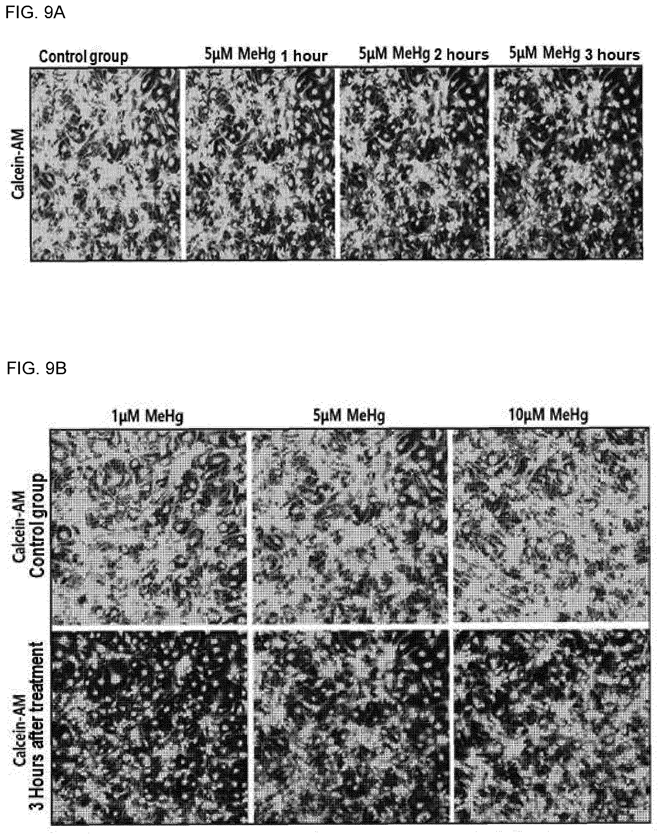

FIG. 9A illustrates confocal microscopic images obtained when astrocytes were cultured for 3 days in the biomimetic nerve chip of the present invention, treated with MeHg, a substance inducing neurotoxicity, at a concentration of 5 µM, and then stained with Calcein-AM to observe viability and morphological changes thereof at 1-hour intervals for 3 hours.
FIG. 9B illustrates confocal microscopic images obtained when astrocytes were cultured for 3 days in the biomimetic nerve chip of the present invention, treated with MeHg, a substance inducing neurotoxicity, at concentrations of 1 µM, 5 µM, and 10 µM, and then stained with Calcein-AM to observe viability and morphological changes thereof according to the concentration after 3 hours.

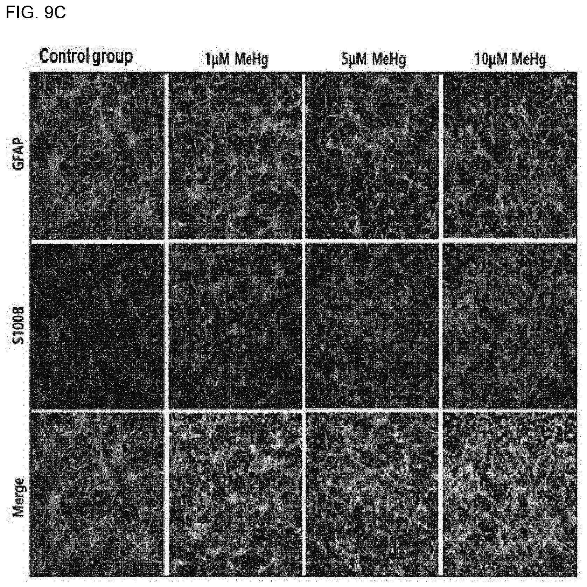

FIG. 9C illustrates confocal microscopic images obtained when astrocytes were cultured for 3 days in the biomimetic nerve chip of the present invention, treated with MeHg, a substance inducing neurotoxicity, at concentrations of 1 µM, 5 µM, and 10 µM, and then stained with the astrocyte-specific markers GFAP and S100B to confirm the identification thereof and observe morphological and internal protein expression changes thereof according to the concentration after 3 hours.

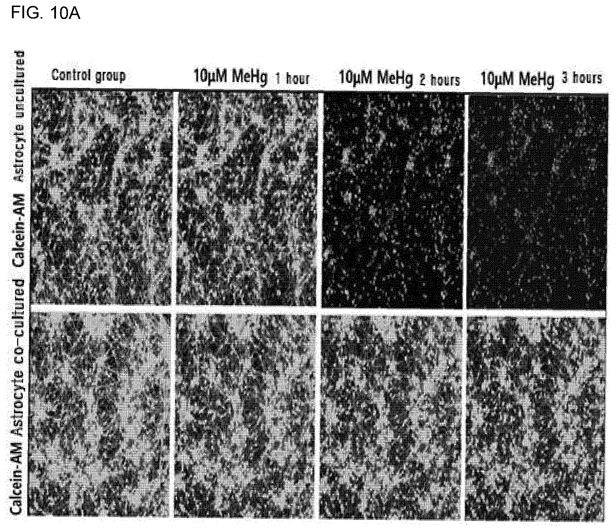

FIG. 10A illustrates confocal microscopic images obtained when under the conditions that only neurons were cultured alone and under the conditions that neurons were co-cultured together with astrocytes, for 3 days in the biomimetic nerve chip of the present invention, the cells were treated with MeHg, a substance inducing neurotoxicity, at a concentration of 10 µM, and then stained with Calcein-AM to confirm the neuroprotective function by astrocytes through viability and morphological changes of neurons at 1-hour intervals for 3 hours.

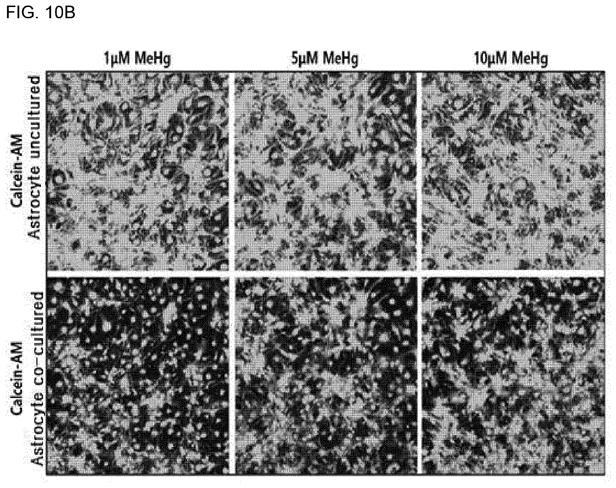

FIG. 10B illustrates confocal microscopic images obtained when under the conditions that only neurons were cultured alone and under the conditions that neurons were co-cultured together with astrocytes, for 3 days in the biomimetic nerve chip of the present invention, the cells were treated with MeHg, a substance inducing neurotoxicity, at concentrations of 1 µM, 5 µM, and 10 µM, and then stained with Calcein-AM to confirm the neuroprotective function by astrocytes through viability and morphological changes of neurons according to the concentration for 3 hours.

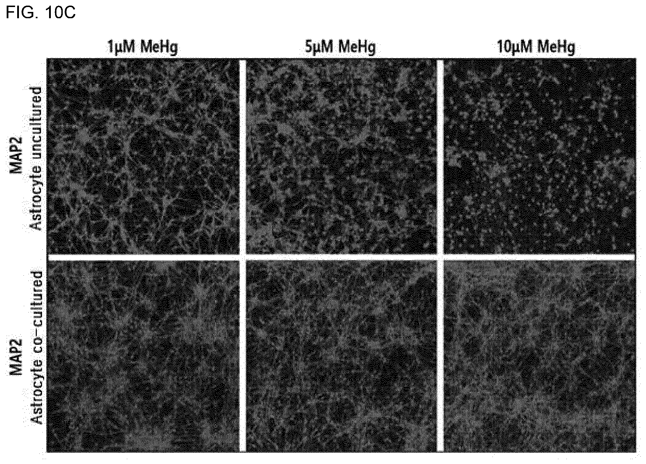

FIG. 10C illustrates confocal microscopic images obtained when under the conditions that only neurons were cultured alone and under the conditions that neurons were co-cultured together with astrocytes, for 3 days in the biomimetic nerve chip of the present invention, the cells were treated with MeHg, a substance inducing neurotoxicity, at concentrations of 1 µM, 5 µM, and 10 µM, and then stained with the neuron-specific marker MAP2 to confirm the neuroprotective function by astrocytes through viability and morphological changes of neurons according to the concentration for 3 hours.

### Best Mode for Carrying Out the Invention

The present inventors configured a biomimetic nerve chip to include astrocytes and neurons constituting the nervous tissue in order to develop a nerve chip capable of mimicking functions of the nervous tissue among organ biomimetic chips.

To achieve this, the present inventors developed a biomimetic nerve chip including elements mimicking, in a structure of the nervous tissue, neurons, astrocytes surrounding the nervous tissue, and capillaries supplying nutrition to the astrocytes, respectively. The element mimicking neurons employed a neuron chamber in which neurons are fixed in the form of the nervous tissue by using an auxiliary component therein; the element mimicking astrocytes employed an astrocyte chamber in which astrocytes are fixed in the form of the astrocytic tissue by using an auxiliary component, extended in the same direction as the neuron chamber, and joined with a portion of the neuron chamber; and the element mimicking the capillaries employed a culture medium channel extended in the same direction as the astrocyte chamber so as to supply the culture medium to the astrocyte chamber and the neuron chamber and joined with a portion of the astrocyte chamber. The cross-communication of the culture medium can be attained through each of the joining portions.

In addition, vascular endothelial cells may be coated on the inner surface of the culture medium channel to increase the similarity between capillaries and the culture medium channel mimicking the capillaries. When the vascular endothelial cells are cultured in ex vivo conditions, the cells are spread thinly, attached, and multiplied on the bottom of the culture container to form a lumen, which is a space between cells. Therefore, when the vascular endothelial cells are injected into the culture medium channel, the cells are spread thinly, attached, and multiplied on the inner surface of the culture medium channel and naturally form a lumen surrounded by the vascular endothelial cells, thereby forming a similar structure to capillaries.

Meanwhile, elements for supplying functional components to the respective chambers and channels are further included. Specifically, in order to supply the neuron chamber with a mixture of neurons as a functional component and an auxiliary component, neuron channels communicating with respective ends of the neuron chamber are provided, and a neuron inlet is provided at one end of the respective provided neuron channels and a neuron outlet is provided at the other end thereof. Next, in order to supply the astrocyte chamber with a mixture of astrocytes as a functional component and an auxiliary component, astrocyte channels communicating with respective ends of the astrocyte chamber are provided, and an astrocyte inlet is provided at one end of the respective provided astrocyte channels and an astrocyte outlet is provided at the other end thereof. Last, in order to supply the culture medium channel with a culture medium as a functional component, the culture medium storage chambers are provided at respective ends of the culture medium channel.

The biomimetic nerve chip developed as described above includes a culture medium supply part, an astrocyte supply part, and a neuron supply part, wherein the culture medium supply part includes: culture medium storage chambers and a culture medium channel; the astrocyte supply part includes an astrocyte inlet, an astrocyte outlet, an astrocyte chamber, and astrocyte channels; and the neuron supply part includes a neuron inlet, a neuron outlet, a neuron chamber, and neuron channels.

Since the biomimetic nerve chip thus developed has a form that structurally mimics the nervous tissue *in vivo,* the chip can be used to pre-test the effect of an externally introduced drug on neurons through astrocytes at a level similar to neurons *in vivo.*

Such a nerve chip that structurally mimics at a level of the nervous tissue to pre-test the effect of a drug on neurons through astrocytes at a level similar to neurons *in vivo* has not been developed until now and was first developed by the present inventors.

In accordance with an aspect of the present invention, there is provided a biomimetic nerve chip for evaluating efficacy and toxicity of a drug, the biomimetic nerve chip including an astrocyte supply part and a neuron supply part for mimicking the nervous tissue, and a culture medium supply part for supplying a culture medium to the astrocyte supply part and the neuron supply part.

Specifically, the biomimetic nerve chip for evaluation efficacy and toxicity of a drug, provided in the present invention, includes:
(a) a culture medium supply part, including, two culture medium storage chambers, and a culture medium channel communicating with each of the chambers;
(b) astrocyte supply part, including, an astrocyte inlet provided at one end thereof, an astrocyte outlet provided at the other end thereof, an astrocyte chamber provided between the astrocyte inlet and the astrocyte outlet and extended in the same direction as the culture medium channel to be joined with a portion of the culture medium channel, wherein a first mixing region for cross-communication of a culture medium is formed in a joining portion between the astrocyte chamber and the culture medium channel, and astrocyte channels provided to communicate between one end of the astrocyte chamber and the astrocyte inlet and between the other end of the astrocyte chamber and the astrocyte outlet, respectively; and
(c) a neuron supply part, including, a neuron inlet provided at one end thereof, a neuron outlet provided at the other end thereof, a neuron chamber provided between the neuron inlet and the neuron outlet and extended in the same direction as the astrocyte chamber to be joined with a portion of the astrocyte chamber, wherein a second mixing region for cross-communication of a culture medium is formed in a joining portion between the neuron chamber and the astrocyte chamber, and neuron channels provided to communicate between one end of the neuron chamber and the neuron inlet and between the other end of the neuron chamber and the neuron outlet, respectively.

A material for the overall structure of the biomimetic nerve chip provided in the present invention is not particularly limited as long as the material does not inhibit or adversely affect the fixation and culture of astrocytes and neurons. In an example, the material may be polycaprolactone (PCL), polydimethylsiloxane (PDMS), polylactic acid (PLA), polyglycolic acid (PGA), polydioxanone (PDO), or the like. In another example, the material may be PDMS that is optically transparent, durable, biocompatible, and flexible, and thus enables easy analysis of the states of astrocytes and neurons through an optical method called motion-tracking.

In order to allow the biomimetic nerve chip manufactured using the material to smoothly perform procedures in which neurons are settled in the neuron chamber, astrocytes are settled in the astrocyte chamber, and a culture medium is introduced into the culture medium channel, a primarily shaped biomimetic nerve chip may be subjected to post-treatment. The post-treatment may be performed on the entirety of the primarily shaped biomimetic nerve chip; the post-treatment may be restrictively performed on the neuron chamber, the astrocyte chamber, and the culture medium channel; and the post-treatment may be restrictively performed on the first mixing region in which the culture medium channel is joined with the astrocyte chamber and the second mixing region in which the astrocyte chamber is joined with the neuron chamber. The post-treatment is not particularly limited, and in an example, the post-treatment may be performed by heating at 60°C to 80°C for 36 to 60 hours. In another example, the post-treatment may be performed by heating at 65°C to 75°C for 44 to 52 hours. In still another example, the post-treatment may be performed by heating in an oven at 70°C for 48 hours.

Hereinafter, each part constituting the biomimetic nerve chip for evaluating efficacy and toxicity of a drug of the present invention will be specifically described.

### 1. Culture medium supply part

A culture medium supply part included in the biomimetic nerve chip provided in the present invention includes two culture medium storage chambers and a culture medium channel.

The culture medium storage chambers store a culture medium to be supplied to the astrocyte chamber and the neuron chamber through the first and second mixing regions and serve to supply the stored culture medium to neurons through the culture medium channel and the astrocyte chamber.

Since both the two culture medium storage chambers serve to supply the culture medium, the culture medium is used to culture astrocytes and neurons after passing through the culture medium channel, and thereafter, the culture medium contaminated with various metabolites and wastes secreted from the astrocytes and neurons is recovered through the astrocyte inlet and outlet and the neuron inlet and outlet.

The culture medium channel serves as a main flow passage of the culture medium where the culture medium flows between the two culture medium storage chambers, wherein a portion of culture medium channel forms a first mixing region together with the astrocyte chamber, and the culture medium is supplied to astrocytes and neurons through the formed first mixing region.

In the present invention, the culture medium channel may be interpreted as an element that mimics capillaries for supplying the blood to neurons. Therefore, the width of the culture medium channel is not particularly limited as long as the channel can mimic the functions of capillaries. In an example, the culture medium channel may have a width of 50 µm to 70 µm. In another example, the culture medium channel may have a width of 55 µm to 65 µm. In still another example, the culture medium channel may have a width of 60 µm.

In addition, vascular endothelial cells may be coated on the inner surface of the culture medium channel to increase the similarity between capillaries and the culture medium channel mimicking the capillaries. For example, when vascular endothelial cells are injected into the culture medium channel, the cells are spread thinly, attached, and multiplied on the inner surface of the culture medium channel and naturally form a lumen surrounded by the vascular endothelial cells, thereby forming a similar structure to capillaries.

For example, in order to form capillary networks adjacent to astrocytes, a vascular endothelial cell line (EA. Hy926) may be loaded into the culture medium channel located at the outermost region in the chip to perform co-culture. In such a case, the vascular endothelial cell line is injected into the channel by using a pipette or a syringe pump in the culture medium storage chamber, and upon completion of the injection, the chip is allowed to stand at 90 degrees or 180 degrees to induce the smooth loading of the cells, by gravity, on the entire surface of the culture medium channel.

### 2. Astrocyte supply part

An astrocyte supply part included in the biomimetic nerve chip provided in the present invention includes an astrocyte inlet, an astrocyte outlet, an astrocyte chamber, and astrocyte channels.

The astrocyte inlet serves to introduce a mixture of astrocytes as a functional component and an auxiliary component to be supplied to the astrocyte chamber, and for example, a nozzle of a syringe loaded with a mixture of astrocytes and an auxiliary component may be connected to the astrocyte inlet.

The astrocytes used in the present invention are not particularly limited as long as the astrocytes can be fixed inside the astrocyte chamber of the biomimetic nerve chip to mimic the functions of astrocytes *in vivo,* and in an example, the astrocytes may be differentiated from stem cells.

The astrocyte mixture may contain astrocytes and an auxiliary component for assisting the flowing and fixation of the astrocytes. The biomimetic nerve chip of the present invention includes astrocytes arranged and present in a fixed form inside the astrocyte chamber included therein, and may additionally include an auxiliary component that assists the injection and fixation of the astrocytes into the astrocyte channels. The auxiliary component may serve as an extracellular matrix (ECM) present in the microenvironment around the astrocytes. The auxiliary component is essential for two-dimensional or three-dimensional cell culture of astrocytes, and maintains the structure and functions of the cultured astrocytes to be similar to those *in vivo.* The auxiliary component is not particularly limited as long as the auxiliary component can assist the introduction and fixation of astrocytes. In an example, the auxiliary component may be a binder for binding astrocytes to the inner surface of the astrocyte chamber, a support agent for allowing astrocytes to form a three-dimensional structure inside the astrocyte chamber and fixing the formed three-dimensional structure, and the like. In another example, the auxiliary component may be Matrigel, which is a kind of binder that binds astrocytes to the inner surface of the astrocyte channels. In still another example, the auxiliary component may be a hydrogel, which is a kind of support agent for allowing astrocytes to form a three-dimensional structure inside the astrocyte channels and fixing the formed three-dimensional structure. In still another example, the auxiliary component may be a collagen hydrogel, an alginate hydrogel, a GelMa hydrogel, or the like, which is a type of hydrogel for allowing astrocytes to form a three-dimensional structure in the astrocyte channels and fixing the formed three-dimensional structure.

When a hydrogel is used as an aspect of the auxiliary component, the mixture of the astrocytes and the hydrogel assists the three-dimensional culture of the astrocytes, and additionally gives fluidity to the astrocytes to allow the astrocytes to move from the astrocyte inlet to the astrocyte chamber through the astrocyte channel. When the hydrogel contained in the mixture is set after the mixture moves to the astrocyte chamber, the astrocytes contained in the set hydrogel may be naturally fixed, and the inherent porosity of the hydrogel enables the delivery of an external culture medium to the astrocytes through the hydrogel, thereby assisting the culture of the fixed astrocytes. The hydrogel may be set under various conditions, and for example, a collagen hydrogel may be set by being left in an incubator for 1 hour or longer to induce the temperature rise; an alginate hydrogel may be set by injecting a CaCl₂ solution, in replacement of the medium, into the culture medium channel; and a GelMa hydrogel may be set by ultraviolet radiation.

The astrocyte outlet may serve to drain the astrocyte mixture that is excessively supplied. The astrocyte outlet may also serve to drain, to the outside, the culture medium supplied through the first mixing region from the culture medium channel and the contaminants, such as various metabolites or wastes secreted from the neurons and supplied from the neuron chamber through the second mixing region. Such drainage of contaminants can be performed through the astrocyte inlet as well as the astrocyte outlet. That is, the astrocyte inlet, of which the role of introducing astrocytes into the astrocyte chamber has been ended, may serve to drain the culture medium and contaminants to the outside. In some cases, the astrocyte inlet or outlet may be connected to an additional storage container.

The astrocyte chamber stores, fixes, and cultures therein the astrocyte mixture supplied through the astrocyte inlet. The astrocyte chamber first serves as a culture container in which the fixed astrocytes are cultured, and second serves as a passage through which the culture medium introduced through the culture medium channel is delivered to neurons fixed in the neuron chamber and various metabolites and wastes secreted from the neurons are drained to the outside.

As described above, the astrocytes injected into the astrocyte chamber may be fixed to the inside of the astrocyte chamber by an auxiliary component injected in the form of a mixture with astrocytes.

In the present invention, the astrocyte chamber may be interpreted as an element that mimics the astrocyte tissue. Therefore, the width of the astrocyte chamber is not particularly limited, and in an example, the width of the astrocyte chamber may be 3 mm to 5 mm × 3 mm to 5 mm horizontally and vertically. In another example, the width of the astrocyte chamber may be 3.5 mm to 4.5 mm × 3.5 mm to 4.5 mm horizontally and vertically. In still another example, the width of the astrocyte chamber may be 4 mm × 4 mm horizontally and vertically.

The astrocyte channels are provided to communicate between one end of the astrocyte chamber and the astrocyte inlet and between the other end of the astrocyte chamber and the astrocyte outlet, respectively. The astrocyte channels serve to deliver the astrocyte mixture, supplied through the astrocyte inlet, to the astrocyte chamber and to deliver the excessive astrocyte mixture in the astrocyte chamber to the astrocyte outlet. In addition, the astrocyte channels may serve as an intermediate passage for draining to the outside an excess of the culture medium introduced from the culture medium channel and various metabolites, wastes, and the like secreted from the neurons.

When the astrocyte mixture is moved to the astrocyte chamber and then the astrocytes contained in the astrocyte mixture are cultured, the culture medium may be delivered to the neuron chamber from the culture medium channel.

In order to allow the culture medium to be delivered from the culture medium channel to the neuron chamber as described above, the astrocyte chamber has a first mixing region formed in a joining portion with the culture medium channel and a second mixing region formed in a joining portion with the neuron chamber. The supply of the culture medium and the recovery of the culture medium used in the culture of neurons are conducted through the first and second mixing regions, and ultimately, the neurons fixed in the neuron chamber can be cultured.

First, the first mixing region may be composed of a portion of the culture medium channel, a portion of the astrocyte chamber joining with the portion of the culture medium channel, and partial block members formed in the joining portion of the channel.

The partial block members serve to partially but not completely open the portion of the culture medium channel and the portion of the astrocyte chamber, which are joined with each other, wherein the partial block members prevent the astrocytes, injected into the astrocyte chamber, from moving to the inside of the culture medium channel prior to fixation of the astrocytes, and allow the culture medium, supplied to the culture medium channel, to move to the astrocytes fixed to the astrocyte chamber through the partially opened sites, ultimately contributing to effective culture of neurons.

The second mixing region may be composed of a portion of the astrocyte chamber, a portion of the neuron chamber joining with a portion of the astrocyte chamber, and partial block members formed in the joining portion.

In such a case, the partial block members may be interpreted as being basically equivalent in shape and function to the partial block members included in the first mixing region. That is, the partial block members included in the second mixing region serve to partially but not completely open the portion of the astrocyte chamber and the portion of the neuron chamber, which are joined with each other, wherein the partial block members prevent the neurons, contained in the neuron mixture injected into the neuron chamber, from moving to the inside of the astrocyte chamber prior to fixation of the neurons and prevent the astrocytes, contained in the astrocyte mixture injected into the astrocyte chamber, from moving to the inside of the neuron chamber prior to fixation of the astrocytes, and allow the culture medium, supplied to the culture medium channel passing through the astrocyte chamber, to move to the neurons fixed to the neuron chamber through the partially opened sites, ultimately contributing to effective culture of neurons.

The partial block members are not particularly limited as long as the partial block members show the above-described functionality. In an example, the partial block members may be micro-post structures that are separated from each other and arranged in a line in a direction of extension of each channel and chamber. The partial block members serve to allow the cross-communication of the culture medium between the portion of the culture medium channel and the portion of the astrocyte chamber and between the portion of the astrocyte chamber and the portion of the neuron chamber, through the separation regions thereof.

Specifically, the micro-post structures serve to partially but not completely open between the portion of the culture medium channel and the portion of the astrocyte chamber and between the portion of the astrocyte chamber and the portion of the neuron chamber, which are joined with each other, through the separation regions thereof, wherein the partial block members prevent the movement of the astrocytes and neurons and allows the cross-communication of the culture medium.

The shape of the micro-post structures is not particularly limited as long as the micro-post structures show the above-described functionality. In an example, the cross-sectional shape of the micro-post structures may be a vertically long hexagonal shape, wherein the arrangement of hexagonal micro-post structures forms bottleneck-structured movement passages between the chambers and the channels to maximize the surface tension of the narrowed portions, thereby facilitating the formation of the boundary of a fluid, and the long vertical shape increases the gap between the formed boundaries of fluids, so that the gap between the boundaries is not easily broken during the injection of the culture medium or the cell mixture, thereby stably preventing the mixing of injected materials.

Therefore, when a culture medium is injected into the culture medium channel, the formation of a boundary is induced by the surface tension at the portions where the movement passages become narrowed by the micro-post structures. In addition, when the astrocyte mixture is injected into the astrocyte chamber, the formation of a boundary is induced by the surface tension at the portions where the movement passages become narrowed by the micro-post structures. Similarly, when the neuron mixture is injected into the neuron chamber, the formation of a boundary is induced by the surface tension at the portions where the movement passages become narrowed by the micro-post structure. As a result, empty gaps are formed between the neuron chamber, the astrocyte chamber, and the culture medium channel on the basis of the micro-post structures, and thus each separation injection can be smoothly performed even without a separation valve or a separation membrane.

The size of the micro-post structures is not particularly limited as long as the micro-post structures show the above-described functionality. In an example, the size of the micro-post structures may be 30 µm to 50 µm and 90 µm to 110 µm horizontally and vertically. In another example, the size of the micro-post structures may be 35 µm to 45 µm and 95 µm to 105 µm horizontally and vertically. In still another example, the size of the micro-post structures may be 40 µm and 100 µm horizontally and vertically. The reason why the horizontal size and vertical size of the micro-post structures are set differently is that the horizontal size shorter than the vertical size allows the culture medium to be introduced into the neuron chamber from the culture medium channel via the astrocyte chamber without interfering with the flow of the culture medium, and such a vertical length sufficiently induces the interval for the boundary forming region, thereby inducing the reproducible formation of empty gaps between the neuron chamber and the astrocyte chamber and between the astrocyte chamber and the culture medium channel.

As used herein, the term "horizontal" corresponds to a surface in a direction parallel to the extension direction of the culture medium channel, the astrocyte chamber, and the neuron chamber among the surfaces of the micro-post erected vertically from the bottom of the astrocyte chamber.

As used herein, the term "vertical" corresponds to a surface in a direction perpendicular to the extension direction of the culture medium channel, the astrocyte chamber, and the neuron chamber among the surfaces of the micro-post erected vertically from the bottom of the astrocyte chamber.

In addition, the separation distance between the micro-post structures is not particularly limited. In an example, the separation distance may be 20 µm to 40 µm. In another example, the separation distance may be 25 µm to 35 µm. In still another example, the separation distance may be 30 µm.

### 3. Neuron supply part

A neuron supply part included in the biomimetic nerve chip provided in the present invention includes a neuron inlet, a neuron outlet, a neuron chamber, and neuron channels.

The neuron inlet serves to introduce a mixture of neuron as a functional component and an auxiliary component to be supplied to the neuron chamber, and for example, a nozzle of a syringe loaded with a mixture of neuron and an auxiliary component may be connected to the neuron inlet.

The neurons used in the present invention are not particularly limited as long as the neurons can be fixed inside the neuron chamber of the biomimetic nerve chip to mimic the functions of neuron *in vivo,* and in an example, the neuron may be differentiated from stem cells.

Similar to as previously described in "2. Astrocyte supply part", the neuron mixture may contain neurons and an auxiliary component for assisting the flowing and fixation of the neurons. The biomimetic nerve chip of the present invention includes neurons arranged and present in a fixed form inside the neuron chamber included therein, and may additionally include an auxiliary component that assists the injection and fixation of the neurons into the neuron channels. The functions and composition of the auxiliary component are as previously described in "2. Astrocyte supply part".

When a hydrogel is used as an aspect of the auxiliary component, the mixture of the neurons and the hydrogel assists the three-dimensional culture of the neurons, and additionally gives fluidity to the neurons to allow the neurons to move from the neuron inlet to the neuron chamber through the neuron channel. When the hydrogel contained in the mixture is set after the mixture moves to the neuron chamber, the neurons contained in the set hydrogel may be naturally fixed, and the inherent porosity of the hydrogel enables the delivery of an external culture medium to the neurons through the hydrogel, thereby assisting the culture of the fixed neurons. The conditions for hydrogel setting is as previously described in "2. Astrocyte supply part".

The neuron chamber may store, fixe, and culture therein the neuron mixture supplied through the neuron inlet, as described above, and may also serve as a culture container in which the fixed neurons are cultured.

As described above, the neurons injected into the neuron chamber may be fixed to the inside of the neuron chamber by an auxiliary component injected in the form of a mixture with astrocytes.

In the present invention, the neuron chamber may be interpreted as an element that mimics neuron, which is the most basic unit of the nervous tissue. Therefore, the width of the neuron chamber is not particularly limited as long as the neuron chamber can perform the fixation and culture of neurons. In an example, the width of the neuron chamber may be 2.5 mm to 4.5 mm × 0.5 mm to 1.5 mm horizontally and vertically. In another example, the width of the neuron chamber may be 3 mm to 4 mm × 0.75 mm to 1.25 mm horizontally and vertically. In still another example, the width of the neuron chamber may be 3.5 mm × 1 mm horizontally and vertically.

The neuron channels are provided to communicate between one end of the neuron chamber and the neuron inlet and between the other end of the neuron chamber and the neuron outlet, respectively. The neuron channels serve to deliver the neuron mixture, supplied through the neuron inlet, to the neuron chamber and to deliver the excessive neuron mixture in the neuron chamber to the neuron outlet. In addition, the neuron channels may serve as an intermediate passage for draining to the outside an excess of the culture medium introduced from the culture medium channel and various metabolites, wastes, and the like secreted from the neurons.

The neuron outlet may serve to drain, to the outside, contaminants, such as various metabolites or wastes secreted from the neurons while the neurons fixed in the neuron chamber are cultured. Such drainage of contaminants can be performed through the neuron inlet as well as the neuron outlet. That is, the neuron inlet, of which the role of introducing neurons into the neuron chamber has been ended through the neuron channel, may serve to drain, to the outside, the contaminants generated while the neurons fixed in the neuron chamber are culture.

The shape of the neuron outlet is not particularly limited as long as the neuron outlet can serve to drain, to the outside, contaminants, such as various metabolites or wastes, secreted from the neurons fixed in the neuron chamber. However, a tube or a nozzle of a syringe may be coupled with the neuron outlet to prevent the contamination of the surrounding environment of neurons due to the contaminants.

The biomimetic nerve chip for evaluating efficacy and toxicity of a drug provided in the present invention basically includes at least one culture medium supply part, one astrocyte supply part, and one neuron supply part, but may include multiple culture medium supply parts, astrocyte supply parts, and neuron supply parts according to the use purpose of the biomimetic nerve chip.

For example, the biomimetic nerve chip may include multiple culture medium supply parts, one astrocyte supply part, and one neuron supply part; one culture medium supply part, multiple astrocyte supply parts, and one neuron supply part; one culture medium supply part, one astrocyte supply part, and multiple neuron supply parts; multiple culture medium supply parts, multiple astrocyte supply parts, and one neuron supply part; multiple culture medium supply parts, one astrocyte supply part, and multiple neuron supply parts; one culture medium supply part, multiple astrocyte supply parts, and multiple neuron supply parts; or multiple culture medium supply parts, multiple astrocyte supply parts, and multiple neuron supply parts.

Especially, when the biomimetic nerve chip includes multiple culture medium supply parts, multiple astrocyte supply parts, and multiple neuron supply parts, multiple first and second mixing regions may be formed, wherein for excluding the interference between the channels, the biomimetic nerve chip is three-dimensionally configured so that multiple same channels are not adjacent to each other or, if adjacent, are completely isolated from each other.

Hereinafter, referring to FIGS. 1 to 5, a structure of the biomimetic nerve chip for evaluating efficacy and toxicity of a drug provided in the present invention will be described in more detail.

The biomimetic nerve chip 1 provided in the present invention is in the form of a structure provided on a slide glass 2. Two sets of astrocyte supply parts each composed of an astrocyte inlet 4, an astrocyte outlet 5, astrocyte channels 9, and an astrocyte chamber 10 are respectively provided above and below a neuron supply part composed of a neuron inlet 6, a neuron outlet 7, neuron channels 11, and a neuron chamber 12. Two sets of culture medium supply parts each composed of culture medium storage chambers 3 and a culture medium channel 8 are respectively provided above and below the astrocyte supply parts.

Portions of the astrocyte chambers 10 form first mixing regions together with portions of the culture medium channels 8 and form second mixing regions together with portions of the neuron chambers 12, wherein the respective mixing regions are differentiated by micro-post structures 13, which are separated from each other and arranged in a line in a direction of extension of each channel.

In accordance with another aspect of the present invention, there is provided a method for evaluating efficacy of a drug on neurons by using the biomimetic nerve chip for evaluating efficacy and toxicity of a drug.

Specifically, the method for evaluating efficacy of a drug on neurons, provided in the present invention, includes: (a) culturing astrocytes contained in an astrocyte chamber and neurons contained in a neuron chamber, the astrocyte chamber and the neuron chamber being included in the biomimetic nerve chip for evaluating efficacy and toxicity of a drug; (b) administering a target drug to the culture medium storage chambers included in the biomimetic nerve chip for evaluating efficacy and toxicity of a drug; and (c) evaluating changes of the cultured astrocytes and neurons.

First, the astrocytes are cultured to form basic morphology, such as a star shape, and the effect of a drug on astrocytes can be evaluated by measuring whether the morphology of the astrocytes is changed. For example, if the astrocytes maintain the star shape thereof after administration of the drug, it may be evaluated that the drug can be used as an agent showing no adverse effect on astrocytes *in vivo.* If the astrocytes aggregate without maintaining the star shape thereof after administration of the drug, it may be evaluated that the drug can serve as an agent inducing the deterioration of astrocytes *in vivo.*

The neurons are cultured to form intact neuron bodies and neurites, which lead to neurite networks, and such neurite networks are an essential component for a main function of neurons, and thus the effect of a drug on neurons can be evaluated by measuring the levels of neurites and neurite networks. For example, if the level of neurites is increased after administration of the drug, it may be evaluated that the drug can be used as an agent promoting nerve formation *in vivo.* If the level of neurites is decreased after administration of the drug, it may be evaluated that the drug can serve as an agent inducing nerve deterioration *in vivo.*

In accordance with still another aspect of the present invention, there is provided a method for evaluating toxicity of a drug on neurons by using the biomimetic nerve chip for evaluating efficacy and toxicity of a drug.

Specifically, the method for evaluating toxicity of a drug on neurons, provided in the present invention, includes: (a) culturing astrocytes contained in an astrocyte chamber and neurons contained in a neuron chamber, the astrocyte chamber and the neuron chamber being included in the biomimetic nerve chip for evaluating efficacy and toxicity of a drug; (b) administering a target drug to the culture medium storage chambers included in the biomimetic nerve chip for evaluating efficacy and toxicity of a drug; and (c) evaluating the presence or absence of a damage to the cultured astrocytes and neurons.

As described above, the use of the biomimetic nerve chip for evaluating efficacy and toxicity of a drug can evaluate, through astrocytes and neurons, whether the supplied drug shows toxicity on astrocytes and neurons.

That is, when a drug is administered to the culture medium storage chamber included in the biomimetic nerve chip, the drug is supplied to the astrocytes together with the culture medium, and then a product modified by the astrocytes may be produced. Therefore, when the neurons are treated with such a modified product, it can be confirmed whether the product modified in the astrocytes, which is not the drug, continuously or rapidly damage neurons, and thus the toxicity of a drug on neurons can be evaluated in a more similar environment to the *in vivo* environment.

The evaluation of toxicity can be performed by using the morphology of astrocytes or the level of neurites as described above. That is, if the astrocytes aggregate without maintaining the star shape thereof or the level of neurites is decreased, after administration of the drug, it may be evaluated that the drug can serve as an agent showing toxicity on astrocytes and neurons *in vivo.*

Still another aspect of the present invention is to provide use of the biomimetic nerve chip for evaluating toxicity of a drug on neurons.

### Mode for Carrying Out the Invention

Hereinafter, the present invention will be described in detail with reference to exemplary embodiments. However, these exemplary embodiments are for illustrative purposes only, and the scope of the present invention is not intended to be limited by these exemplary embodiments.

### Example 1: Securing of human stem cell-based neurons and astrocytes

Neurons differentiated from human stem cells (hPSCs) were secured through neurons (Neurosight-S) by NEXEL. A stock was thawed and injected immediately into the chip. The neurons were prepared at an appropriate concentration for injection of 15×10⁴ cells/6 µL, loaded into the neuron inlet included in the nerve chip of the present invention, and injected into the neuron chamber passing through the neuron channel. The bottom of the neuron chamber was coated with Matrigel, which was three times more concentrated than usual, for stable attachment in the chip, and the neurons were cultured in Neurosight-S medium supplemented with Supplement (100X) for 3 days during which neurites can be formed.

Astrocytes, differentiated from human stem cells (hPSCs), were secured by direct differentiation. Human stem cells (hPSCs) were maintained through culture and passage by using TeSr-E8 medium in a Matrigel-coated culture dish, and for differentiation into neurons, neural progenitor cells (NPCs) were first prepared. The stem cell colonies were detached using Collagen IV, and then embryoid bodies (EBs) were formed using DMEM/F12 medium containing 20% knock out serum replacement, 1% nonessential amino acids, 55 µM β-mercaptoethanol, 10 µM SB431542, and 5 µM DMH1, and then again attached to a Matrigel-coated culture dish to form neural rosettes in DMEM/F12 medium containing 1% N2 supplement, 2.5 µL/mL insulin, and 20 ng/mL basic fibroblast growth factor. Only the center of the rosettes was separated using a thinly drawn Pasteur pipette, and then cultured in DMEM/F12 medium containing 1% N2 supplement, 20 ng/mL basic fibroblast growth factor, and 20 ng/mL epidermal growth factor to secure NPCs. The secured NPCs were again attached to a Matrigel-coated culture dish at a density of 2.8×10³ cells/cm, and cultured in DMEM/F12 medium containing 1% N2 supplement, 2% vitamin A-removed B27 supplement, 10 ng/mL bone morphogenetic protein-4, 10 ng/mL ciliary neurotrophic factor, and 10 ng/mL leukemia inhibitory factor to thereby obtain finally differentiated astrocytes.

The astrocytes thus secured were prepared at an appropriate concentration for injection of 15×10⁴ cells/60 µL, and 30 µL was loaded into each astrocyte inlet included in the nerve chip of the present invention and injected into two astrocyte chambers passing through astrocyte channels by using a syringe pump. Likewise, the bottom of the astrocyte chambers was coated with Matrigel, which was three times more concentrated than usual, for stable attachment in the chip, and co-cultured along with neurons in Neurosight-S medium for 3 days. It was confirmed that there was no abnormality when the astrocytes were cultured in such media. All cells were stored in a humidified incubator at 37°C and 5% CO₂.

### Example 2: Fabrication of biomimetic nerve chip

The biomimetic nerve chip was fabricated using a polydimethylsiloxane (PDMS) elastic polymer and a slide glass. PDMS, which is an optically transparent and highly durable polymer, does not harm the cells because of being bio-friendly properties and facilitates the analysis of the states of neurons and astrocytes against a toxic substance through an optical method since it is a transparent and flexible material.

The biomimetic nerve chip was designed by an AutoCAD program in consideration of the following five physiological characteristics of the nervous system in order to realize the microenvironment adjacent to the *in vivo* neurons inside the chip: 1) the nervous system consists of two types of cells, neurons and glia; 2) The glia-to-neuron ratio (GNR) in the human brain is about 1:1; 3) nutrients are delivered from blood vessels to neurons through astrocytes by the glia-astrocyte interaction in a metabolic aspect; 4) neurons are usually exposed under the flow of interstitial fluid (ISF), which serves as an intercellular communication channel within the human brain; and 5) neurons are protected by astrocytes from MeHg toxicity.

A film mask for manufacturing a 75 µm-high mold was fabricated, and a mold was manufactured using the film mask, SU-8 (MICROCHEM) as photoresist (PR), and photolithography, so that a PDMS-based microfluidic device with an in-chip microstructure was fabricated. A specific method was as follows:
About 1.5 mL of the photoresist was dropped on a silicon wafer, and then evenly coated (PR coating) to a thickness of 75 µm by using a spin coater. Then, the wafer was placed on a hot plate heated to 80°C and subjected to soft baking for about 10 minutes to remove moisture and allow the photoresist to adhere strongly to the surface of the wafer, thereby preventing a portion exposed to UV light from being torn off during PR developing. The film mask was aligned above the wafer coated with the photoresist, and then subjected to ultraviolet radiation by using a UV exposable mask aligner. It was confirmed that in a negative photoresist subjected to UV exposure as such, a portion exposed to UV light was set more.

Thereafter, PR developing was conducted by immersion in a photoresist developer (SU-8 developer, MICROCHEM) for about 1 minute and 30 seconds, thereby selectively removing a portion not exposed to UV light. Finally, the wafer was washed with deionized water (Dl water) and dried with nitrogen, and then the wafer was subjected to post exposure baking by placing the wafer at 80°C for about 10 minutes to thereby obtain a final mold with a microstructure patterned.

On the mold that has been subjected PR patterning, a PDMS silicone base elastomer and a curing agent (SYLGARD 184 silicone elastomer kit, Dow Corning) mixed at a ratio of 10:1 were poured onto the SU-8 mold, and set by heating at 80°C overnight. After separated from the mold, PDMS was cut according to the size of the chip, and then punched out by using cylindrical biopsy punchers with diameters of 1.5 mm and 6 mm for neuron inlets and outlets, astrocyte inlets and outlets, and culture medium storage chambers according to the sizes thereof.

As for the fabricated microfluidic device, the PDMS-based microfluidic device was immersed in a sonicator containing isopropyl alcohol and washed by sonication for 60 seconds, and then completely evaporated by a nitrogen air-gum. The PDMS-based microfluidic device that has been washed and a slide glass were dried in a dry oven at 70°C for 5 minutes and then sterilized by exposure to UV light for 3 hours. Finally, the PDMS-based microfluidic device and the slide glass were placed in a plasma processing system (CUTE, FEMTO Science) and allowed to adhere to each other through oxygen plasma treatment to thereby fabricate a biomimetic nerve chip.

The biomimetic nerve chip obtained by adhering was placed in a 35 mm petri dish and, for enhancing the adhesion resulting from the plasma treatment, was heated on a hot plate heated to 80°C for 15 minutes, and then subjected to a post-treatment process for subsequent injection in an oven at 70°C for 48 hours. Such post-treatment process was performed to selectively inject a corresponding component into a desired channel.

The size of the biomimetic nerve chip fabricated as described above was 3.5 cm and 1.9 cm horizontally and vertically, and the heights of all microstructures in the chip were set to 75 µm.

As shown in FIG. 1, the main inner microstructures of the nerve chip were configured of the neuron chamber located in the center, the two astrocyte chambers adjacent to the neuron chamber, the two culture medium channels located at the outermost region, and 4 rows of arrangement of micro-post structures located in the spaces between the chambers and the channels. Through this placement, the flow of the culture medium of passing through the astrocyte chambers from the culture medium channels located at the sides and finally reaching the neuron chambers was considered similarly that nutrients are delivered from blood vessels to neurons through astrocytes in the *in vivo* microenvironment.

As for the actual sizes of the prepared cells, astrocytes are larger than neurons, and thus through several culture experiments, the ratio of the area of the neuron chamber to the total area of the two astrocyte chambers is made in proportional to the cell sizes, so that the injection is performed at a ratio of 1:1, which is similar to the ratio of neurons and glia *in vivo,* and therefore, the neuron chamber was set to be 3.5 mm × 1 mm horizontally and vertically, and each of the astrocyte chambers was set to 4 mm × 4 mm horizontally and vertically.

In addition, in order to implement an environment in which the culture medium flows at a low speed similar to the flow of the brain interstitial fluid (ISF) *in vivo* while minimizing the shear stress caused by a high-speed culture medium flow that may occur in a cell chamber when the culture medium was injected directly through the channels to inject each type of cells, like in previous studies, long culture medium channels capable of inducing long-distance movement of the culture medium from the culture medium storage chambers were disposed at the outermost region, and in order to set the culture medium channels to a similar size to blood vessels *in vivo,* the width of the culture medium channels was set to 60 µm.

In order to selectively inject a specific type of cells into a desired space within the chip, a microvalve capable of physically separating the space from other spaces is generally needed. However, most microvalves require cumbersome operation methods and external driving force, and it is very difficult to reproducibly perform separation injection into adjacent chambers by using only micro-post structures known through the previous studies.

In the present invention, as shown in FIG. 5, micro-post structures with a vertically long hexagonal shape of 40 µm × 100 µm horizontally and vertically were configured in four arrangements between the chambers and the channels, and the respective micro-post were separated from each other at a distance of 30 µm.

The first reason for designing the micro-post structures in a vertically long hexagon shape is that the arrangements of micro-posts with a hexagonal shape lead to bottleneck-structured movement passages between the chambers and channels, so that the surface tension at narrowed portions of the movement passages is maximized to easily form boundaries of the fluids. The second reason is that the vertically long shape increases the gap between the formed boundaries of the fluids, wherein the gap between the boundaries during cell injection is not easily broken during cell injection and thus can stably protect fluid mixing.

As shown in FIG. 6, it was examined, by injecting food colorings into each of the chambers and channels by using a syringe pump, whether the fluids could be injected into the adjacent neuron chamber, astrocyte chambers, and culture medium channels without fluid mixing even without the presence of microvalves or separation membranes. It was confirmed that empty gaps were well formed by the micro-post structures between the chambers and the channels.

The diameter of each of the inlets and outlets for injecting neurons and astrocytes was 1.5 mm, and the diameter of the culture medium storage chambers was set to 6 mm.

### Example 3: Construction of bio-similar environment using elements in biomimetic nerve chip

First, as mentioned above, for stable attachment of cells in the chip, nozzles of syringes containing Matrigel prepared by concentration three times more than usual was connected to the astrocyte inlets connected to the astrocyte chambers and channels, and then separation injection was performed using a syringe pump (NE-1000, NEWERA). Similarly, a nozzle of a syringe containing Matrigel was connected to the neuron inlet connected to the neuron chamber and channels, and then separation injection was performed using a syringe pump. Successful separation injection was identified by confirming that Matrigel forms boundaries at the narrowed portions of bottleneck structures formed by the micro-post structures. Thereafter, coating was conducted in a humidified incubator at 37°C and 5% CO₂ for about 1 hour, and upon completion of the coating, the neuron chamber and the astrocyte chambers were washed with dPBS.

The injection of cells was conversely conducted. Human stem cell-derived neurons (Neurosight-S) were preferentially injected, wherein the neurons were prepared at an appropriate concentration for injection of 15×10⁴ cells/6 µL, load into the neuron inlet, and injected into the neuron chamber. Human stem cell-derived astrocytes were prepared at an appropriate concentration for injection of 15×10⁴ cells/6 µL, and nozzles of syringes each carrying 30 µL were connected to the astrocyte inlets, and the astrocytes were injected into the two astrocyte chambers by using a syringe pump.

Thereafter, 100 µL of a culture medium was loaded into two culture medium storage chambers, and the culture medium channels located on both sides were filled with a culture medium by using a pipette. Then, 100 µL of a culture medium was loaded into the remaining two culture medium storage chambers to connect the flow of the culture medium between the culture medium storage chambers. In such case, it was confirmed that the culture medium formed boundaries at the narrowed portions of the bottleneck structures formed by the micro-post structures. The gaps temporally formed during separation injection into the nerve chip disappear naturally when the separation injection of the cells was completed. When all of the gaps inside the chip disappeared, the cells were cultured in a humidified incubator at 37°C and 5% CO₂.

The culture medium was exchanged with a fresh culture medium every day to provide a continuous medium flow and fresh nutrients, and the cells were cultured while wastes were removed by draining the culture medium discharged to the neuron inlet and outlet and the astrocyte inlets and outlets during the culture medium exchange.

As can be seen in FIG. 7A, the neurons in the chip generated neurites as soon as the neurons were attached, and formed neurite networks at about 3 days of culture. The astrocytes also generated endfeet as soon as the astrocytes were attached, and were uniformly spread to all places in the astrocyte chambers through natural cell movement.

Therefore, the optimal period of culture was set to 3 days before toxicity evaluation, and it was confirmed, through the above-described separation injection of cells, that only corresponding cells were cultured in a corresponding chamber on the basis of the micro-post structures.

Additionally, as shown in FIG. 7B, the neurons were labeled with the selective marker Tuj1 and the mature marker MAP2, and the astrocytes were labeled with the selective marker GFAP and the mature maker S100B through immunostaining, and it was confirmed that healthy neurons were cultured in the neuron chamber, and healthy astrocytes were cultured in the astrocyte chamber.

### Example 4: Drug toxicity testing using biomimetic nerve chip

It was assessed whether neurotoxicity can be evaluated by a noticeable biomimetic nerve chip of the present invention in which an *in vivo* environment similar to physiological characteristics of the nervous system was constructed. First, methylmercury chloride (MeHg), a representative environmental toxic substance known to pass through the brain barrier regardless of a structure of the brain barrier and to have neurotoxicity, was selected as a toxic substance for treatment. The neurotoxicity evaluation was conducted by treating a chip having only neurons cultured and a chip having neurons and astrocytes co-cultured, with MeHg at various concentrations (0 µM, 1 µM, 5 µM, 10 µM) for 3 hours and then examining the cell response changes according to the MeHg time and concentration through real-time live cell imaging and immunocytochemistry, and then the functionality of the biomimetic nerve chip as a drug toxicity evaluation platform was assessed (FIGS. 8 to 10).

As can be seen in FIG. 8A, in order to examine the response of neurons according to the time due to the toxicity of MeHg by using the biomimetic nerve chip, the treatment with 5 µM MeHg was conducted under the condition in which only neurons were cultured, and cell viability was evaluated in real time by using Calcein-AM, a marker capable of staining only living cells. Most of the neurons in the control group formed neurite networks while having intact cell bodies and neurites. After the treatment with 5 µM MeHg, the neurites maintained the state thereof with deterioration little by little without any significant change until 2 hours after the treatment with MeHg, but after the last 3 hours of treatment, the neurites almost completely disappeared, leaving only fragmented fragments and only a form of cell bodies. These results indicated that the survival of neurons was decreased due to neurotoxicity as much as the time the neurons were exposed to MeHg.

As shown in FIG. 8B, the level of neurotoxicity according to the concentration of MeHg was examined by using the nerve chip under the same culture conditions without astrocytes. The MeHg treatment concentrations were set to 1 µM, 5 µM, and 10 µM, and similarly, the viability of neurons was examined in real time through Calcein-AM. 3 hours after MeHg treatment at the lowest concentration of 1 µM, some neurites endured neurotoxicity of MeHg without damage to maintain the state thereof. 3 hours after the treatment at 5 µM, neurites disappeared and only the cell bodies remained. 3 hours after the treatment at 10 µM, even the fluorescence signal of the remaining cell bodies was greatly reduced. These results indicated that the level of neurotoxicity was changed in proportion to the concentration, leading to a change in the viability of neurons.

The cell types can be distinguished by morphological characteristics of cells, but Calcein-AM targets all living cells regardless of the type of cells and thus cannot clearly identify the types of cells. The present inventors re-examined whether the neurons were damaged at the same extent by MeHg, through immunostaining using Tuj1 and MAP2 antibodies targeting only neurons (FIG. 8C).

As shown in FIG. 8C, as a result of immunostaining 3 hours after the treatment with MeHg at concentrations of 1 µM, 5 µM, and 10 µM, the damage to cells increased as the concentration increased, as in the results confirmed using Calcein-AM. These results indicated that the changes of neurons due to MeHg toxicity could be reproducibly observed by using the nerve chip.

As the toxicity of MeHg to neurons was observed as described above, and now, the changes of astrocytes due to the toxicity of MeHg were observed in the same manner (FIGS. 9A and 9B).

As shown in FIGS. 9A and 9B, in order to examine the response of astrocytes according to time and concentration changes due to toxicity of MeHg by using the biomimetic nerve chip, MeHg treatment was conducted under the condition in which astrocytes were cultured, and cell viability was evaluated in real time by using Calcein-AM. As a result, morphological changes of astrocytes due to cell aggregation were observed over time, but no decrease in cell viability was observed. There was also no significant difference in the degree of morphological change due to cell aggregation regardless of the concentration.

On the other hand, several reports demonstrated a correlation between brain injury and the S100B level in cerebrospinal fluid (CSF), and some of the reports showed that an increase in S100B was observed in the CSF of neonatal stage mice exposed to MeHg. On the basis of the assertion that this S100B increase is associated with CNS damage, immunostaining was performed using GFAP and S100B, which were used as markers for brain damage due to metal-induced neurotoxicity, in order to identify astrocytes and examine the response of astrocytes in the MeHg detection (FIG. 9C).

As shown in FIG. 9C, the viability of astrocytes was not changed as before, and the morphological changes due to cell aggregation were also re-confirmed. The most interesting result was that the expression level of GFAP was inversely proportional to the expression level of S100B, and in other words, as the concentration of MeHg increased, the S100B signal increased but the GFAP signal decreased, similar to *in vivo* conditions. Most of the GFAP-positive astrocytes had a basic morphology such as a star shape, and in contrast, most of the S100B-positive cells had a crushed form by the loss of endfeet due to cell aggregation. These results provided a strong basis for asserting that the nerve chip of the present invention is a multi-purpose platform in which the response of a particular type of cells, such as an *in vivo* protein expression pattern, can be re-produced.

Finally, it was examined whether there was a neuroprotective function from the neurotoxicity of MeHg depending on the presence or absence of astrocytes inside the biomimetic nerve chip. MeHg treatment was conducted under two conditions in which astrocytes were cultured alone and neurons and astrocytes were co-cultured, and the cell viability was evaluated in real time according to the time and concentration changes by using Calcein-AM (FIGS. 10A to 10C).

As shown in FIG. 10A, for the most reliable comparison of results with respect to time-dependent changes, real-time changes were compared after the treatment with a concentration of 10 µM. In the neurons cultured alone, most of the Calcein-AM signals started to rapidly decrease 2 hours after MeHg treatment, but in the astrocytes co-cultured, no significant changes were observed, except for a few strands of neurites that partially disappeared, until 3 hours after treatment.

As shown in FIGS. 10B and 10C, as for comparison of the response according to the concentration change, the Calcein-AM signal and the MAP2 signal were maintained without any decrease, regardless of the concentration, in the presence of astrocytes, when compared with the results under neurons cultured alone. It was therefore confirmed that the mechanisms of astrocyte-mediated neuroprotection against MeHg toxicity can be sufficiently studied through the nerve chip.

The above results indicate that the biomimetic nerve chip provided in the present invention could be assessed as a system capable of deriving reliable results in performing the neurotoxicity evaluation.

While the present invention has been described with reference to the particular illustrative embodiments, a person skilled in the art to which the present invention pertains can understand that the present invention may be embodied in other specific forms without departing from the technical spirit or essential characteristics thereof. Therefore, the examples described above should be construed as being exemplified and not limiting the present invention. The scope of the present invention is not defined by the detailed description as set forth above but by the accompanying claims of the invention, and it should also be understood that all changes or modifications derived from the definitions and scopes of the claims and their equivalents fall within the scope of the invention.

### Explanation of reference numerals

1: PDMS-based microfluidic device
2: Slide glass
3: Culture medium storage chamber
4: Astrocyte inlet
5: Astrocyte outlet
6: Neuron inlet
7: Neuron outlet
8: Culture medium channel
9: Astrocyte channel
10: Astrocyte chamber
11: Neutron channel
12: Neuron chamber
13: Micro-spot structure

## Claims

1. A biomimetic nerve chip for evaluating efficacy and toxicity of a drug, the biomimetic nerve chip comprising:
(a) a culture medium supply part, comprising,
two culture medium storage chambers, and
a culture medium channel communicating with each of the chambers;
(b) an astrocyte supply part, comprising,
an astrocyte inlet provided at one end thereof,
an astrocyte outlet provided at the other end thereof,
an astrocyte chamber provided between the astrocyte inlet and the astrocyte outlet and extended in the same direction as the culture medium channel to be joined with a portion of the culture medium channel, wherein a first mixing region for cross-communication of a culture medium is formed in a joining portion between the astrocyte chamber and the culture medium channel, and
astrocyte channels provided to communicate between one end of the astrocyte chamber and the astrocyte inlet and between the other end of the astrocyte chamber and the astrocyte outlet, respectively; and
(c) a neuron supply part, comprising
a neuron inlet provided at one end thereof,
a neuron outlet provided at the other end thereof,
a neuron chamber provided between the neuron inlet and the neuron outlet and extended in the same direction as the astrocyte chamber to be joined with a portion of the astrocyte chamber, wherein a second mixing region for cross-communication of a culture medium is formed in a joining portion between the neuron chamber and the astrocyte chamber, and
neuron channels provided to communicate between one end of the neuron chamber and the neuron inlet and between the other end of the neuron chamber and the neuron outlet, respectively.

2. The biomimetic nerve chip of claim 1, wherein the culture medium storage chambers store therein a culture medium to be supplied to the astrocyte chamber and the neuron chamber through the first and second mixing regions and serve to supply the stored culture medium to neurons through the culture medium channel and the astrocyte chamber.

3. The biomimetic nerve chip of claim 1, wherein the culture medium channel serves as a main flow passage of a culture medium where the culture medium flows between the two culture medium storage chambers.

4. The biomimetic nerve chip of claim 1, wherein an inner surface of the culture medium channel is coated with vascular endothelial cells.

5. The biomimetic nerve chip of claim 1, wherein the astrocyte chamber stores, fixes, and cultures therein a mixture of astrocytes and an auxiliary component supplied through the astrocyte inlet, serves as a culture container in which the fixed astrocytes are cultured, and serves as a passage through which the culture medium introduced through the culture medium channel is delivered to neurons fixed in the neuron chamber and various metabolites and wastes secreted from the neurons are drained to the outside.

6. The biomimetic nerve chip of claim 1, wherein the neuron chamber stores, fixes, and cultures therein a mixture of neurons and an auxiliary component supplied through the neuron inlet and serves as a culture container in which the fixed neurons are cultured.

7. The biomimetic nerve chip of claim 5 or 6, wherein the auxiliary component is Matrigel, a collagen hydrogel, an alginate hydrogel, or a GelMa hydrogel.

8. The biomimetic nerve chip of claim 1, wherein the first mixing region is composed of a portion of the culture medium channel, a portion of the astrocyte chamber joined with the portion of the culture medium channel, and partial block members formed in the joining portion.

9. The biomimetic nerve chip of claim 1, wherein the second mixing region is composed of a portion of the astrocyte chamber, a portion of the neuron chamber joined with the portion of the astrocyte chamber, and partial block members formed in the joining portion.

10. The biomimetic nerve chip of claim 8 or 9, wherein the partial block members are micro-post structures separated from each other and arranged in a line in a direction of extension of the channels and chambers.

11. The biomimetic nerve chip of claim 10, wherein the micro-post structures allow cross-communication of a culture medium, through the separated regions thereof, between the portion of the culture medium channel and the portion of the astrocyte chamber joining with the portion of the culture medium channel and between the portion of the astrocyte chamber and the portion of the neuron chamber joining with the portion of the astrocyte chamber.

12. A method for evaluating efficacy of a drug on neurons, the method comprising:
(a) culturing astrocytes contained in an astrocyte chamber and neurons contained in a neuron chamber, the astrocyte chamber and the neuron chamber being included in the biomimetic nerve chip for evaluating efficacy and toxicity of a drug of any one of claims 1 to 11;
(b) administering a target drug to the culture medium storage chambers included in the biomimetic nerve chip for evaluating efficacy and toxicity of a drug; and
(c) evaluating changes of the cultured astrocytes and neurons.

13. The method of claim 12, wherein in step (c), the drug is evaluated as an agent that promotes the formation of nerve *in vivo* when the level of neurites formed from the neurons is increased.

14. A method for evaluating toxicity of a drug on neurons, the method comprising:
(a) culturing astrocytes contained in an astrocyte chamber and neurons contained in a neuron chamber, the astrocyte chamber and the neuron chamber being included in the biomimetic nerve chip for evaluating efficacy and toxicity of a drug of any one of claims 1 to 11;
(b) administering a target drug to the culture medium storage chambers included in the biomimetic nerve chip for evaluating efficacy and toxicity of a drug; and
(c) evaluating the presence or absence of a damage to the cultured astrocytes and neurons.

15. The method of claim 14, wherein in step (c), the drug is evaluated as an agent that shows toxicity on neurons *in vivo* when the level of neurites formed from the neurons is decreased.

16. The method of claim 14, wherein in step (c), the drug is evaluated as an agent that shows toxicity on neurons *in vivo* when the astrocytes aggregate without maintaining a star shape.
